# EUROPÄISCHE PATENTSCHRIFT

(11) **EP 1 256 627 B1**
(45) Veröffentlichungstag und Bekanntmachung des Hinweises auf die Patenterteilung: **09.11.2005**
(21) Anmeldenummer: 02009890.1
(22) Anmeldetag: 02.05.2002
(51) Int. Cl.: C12N 15/10

(54) **Verwendung von Alkanen zur kontaminationsfreien Reinigung bzw. Trennung von Biopolymeren**
Use of alkanes for contamination-free purification or separation of biopolymers
Utilisation d'alcanes pour la séparation et purification de biopolymers

(30) Priorität: 11.05.2001 DE 10122990
(43) Veröffentlichungstag der Anmeldung: 13.11.2002
(73) Patentinhaber: QIAGEN GmbH, 40724 Hilden (DE)
(72) Erfinder: Rothmann, Thomas, 40764 Langenfeld (DE); Fabis, Roland, 42781 Haan (DE); Schäfer, Andreas, 51381 Leverkusen (DE); Dorit Menzel, Sabine, 40237 Düsseldorf (DE); Nguyen, Thi My Chi, 40472 Düsseldorf (DE)
(74) Vertreter: Zimmermann, Gerd Heinrich

(56) Entgegenhaltungen:
- DE-A- 19 746 874
- US-A- 4 902 481
- US-A- 5 846 493
- "QIAquick Spin Handbook" April 2000 (2000-04) , QIAGEN XP002237650 "Punkt 1" * Seite 19 - Seite 20 *

## Beschreibung

Die vorliegende Erfindung betrifft die Verwendung von Kohlenwasserstoffen zur Vermeidung von Kreuzkontaminationen bei der Elution von Flüssigkeiten aus Vorrats- bzw. Probenaufnahmegefäßen.

Insbesondere betrifft die vorliegende Erfindung die Verwendung von verzweigten oder unverzweigten Kohlenwasserstoffen mit 5 bis 20 Kohlenstoffatomen zurkontaminationsfreien Trennung und oder Reinigung von Biopolymeren, insbesondere von Nukleinsäuren und Proteinen aus pflanzlichen, tierischen oder menschlichen Zellen oder Zellteile beinhaltenden Flüssigkeiten.

Zum Trennen von Flüssigkeitsproben in ihre einzelnen Bestandteile, Reinigen bestimmter Komponenten der Flüssigkeitsprobe oder auch Filtern der Flüssigkeitsproben wird die Flüssigkeit in ein Probenaufnahmegefäß eingebracht (pipettiert), wo sie eine Filterschicht (Filterpapier, Glasfritte, Membran oder Material mit selektiver Adsorptionseigenschaft) durchdringt und über eine Auslaßöffnung gegebenenfalls tropfenweise in ein mit einem bestimmten Abstand unter dem Probenaufnahmegefäß angeordnetes Auffangbehältnis gelangt. Das Probenaufnahmegefäß und das Auffangbehältnis sind im allgemeinen röhrenförmig ausgebildet, wobei die Filterschicht auf der mit der Auslaßöffnung versehenen stirnseitigen Bodenwand des Probenaufnahmegefäßes aufliegt. Die Auslaßöffnung hat einen Durchmesser von einigen wenigen 1/10 mm. mehrere derartige Probeaufnahmegefäße sind nebeneinander in Reihen und Spalten angeordnet und über eine Trägerplatte miteinander verbunden. Das Durchdringen der Filterschicht erfolgt insbesondere durch Ansaugen der Flüssigkeitsprobe infolge eines Unterdrucks. Zu diesem Zweck wird eine einem Unterdruck aussetzbare Kammer mit der die Probenaufnahmegefäße haltenden Trägerplatte luftdicht verschlossen. Innerhalb der Kammer befinden sich die den Probenaufnahmegefäßen zugeordneten Auffangbehältnisse, die in einem Gestell untergebracht und gehalten sind. Derartige Apparaturen werden beispielsweise in medizinisch-technischen Labors zum gleichzeitigen Filtern von einer Vielzahl von Flüssigkeitsproben mehrerer Patienten eingesetzt.

Aus US-PS 47 77 021 und 44 27 415 sind derartige Vorrichtungen bekannt. Beiden bekannten Vorrichtungen gemeinsam ist der Umstand, daß die die Filterschichten durchdringenden Flüssigkeitstropfen der Proben in ein gemeinsames wannenartiges Auffangbehältnis gelangen, welches Teil der Vakuum- oder Unterdruckkammer ist, die durch die einzelnen matrixförmig angeordneten Probenaufnahmegefäße miteinander verbindenden Trägerplatte verschlossen ist. Bei den bekannten Vorrichtungen zum Trennen von Flüssigkeitsproben ist der von bzw. in der Filterschicht zurückgehaltenen Bestandteil der Probe von Interesse für die nachfolgenden Untersuchungen. Die die Filterschichten durchdringende Flüssigkeit ist für die weitere Analyse "verloren". Für die Trennung bei der chemischen oder der Biopolymerpräparation von Proben ist es jedoch erforderlich, die die Filterschicht durchdringende oder aus der Filterschicht durch Aufbringen von Lösungsmittel ausgewaschenen bzw. gelösten Probenbestandteile einzeln oder getrennt voneinander auffangen zu können.

Aus der US-PS 49 02 481 ist eine Vorrichtung bekannt, bei der in die Unterdruckkammer ein Einsatzstück mit mehreren matrixförmig angeordneten Auffangbehältnissen eingesetzt ist, die jeweils unterhalb der Probenaufnahmegefäße angeordnet sind. Die die Probenaufnahmegefäße miteinander verbindende Trägerplatte befindet sich an den oberen Enden der Probenaufnahmegefäße. Die Probenaufnahmegefäße, die rohrförmig sind, wobei das untere stirnseitige Ende durch die Filterschicht verschlossen ist, sind in eine auf der Oberseite mit aufstehenden geschlossenen Rädern versehenen Steckplatte eingesteckt, die mit mehreren Löchern versehen ist. Auf der Unterseite schließt sich an jedes Loch ein relativ kurzes Auslaßröhrchen an, das eine gestufte Außenumfangsfläche aufweist. Ferner sind auf der Unterseite pro Auslaßröhrchen ein dieses umgebender geschlossener Rand angeformt, wobei der Durchmesser dieser Ringränder gleich den Durchmessern der Auffangbehältnisse ist, die mit Abstand unterhalb der geschlossenen Ränder auf der Unterseite der Steckplatte angeordnet sind. Die Auslaßröhrchen reichen dabei nicht bis in die gehörigen Auffangbehältnisse hinein.

Die einzelnen Auffangbehältnisse der Vorrichtung nach US-PS 49 02 481 haben lediglich einen geringen Abstand voneinander. Aufgrund des Abstandes der Auffangbehältnisse von den Probenaufnahmegefäßen besteht die Gefahr, daß Teile eines Flüssigkeitstropfens, der von dem unter einem Probenaufnahmegefäß angeordneten Auffangbehältnis aufgenommen werden soll, in ein benachbartes Auffangbehältnis gelangt und dessen "Filtrat" kontaminiert. Ferner ist die Tropfenbildung bei der bekannten Vorrichtung nach US-PS 49 02 481 nicht gleichförmig, insbesondere dann ungleichförmig, wenn die Unterdruckkammer kurzzeitig belüftet wird, um den in ihr untergebrachten Satz von Auffangbehältnissen gegen einen neuen auszuwechseln. Bei der Belüftung der Unterdruckkammer wird nämlich die Unterseite der Steckplatte mit Tropfenflüssigkeit benetzt. Bei anschließender Erzeugung eines Unterdrucks bilden sich nun relativ große Tropfen, da die angesaugte Flüssigkeit aufgrund der Benetzung der Unterseite sich entlang dieser ausgebreitet. Hierbei kann der Tropfen bis zum Ringrand reichen, wo er über den Spalt zwischen Ringrand und Auffangbehältnis abgesaugt wird. Die Flüssigkeit gelangt also nicht in das gewünschte Auffangbehältnis, sondern unter Umständen in ein benachbartes Auffangbehältnis (Kontamination) bzw. läuft außen an den Auffangbehältnissen entlang. Kontaminationen der von den Auffangbehältnissen aufgefangenen Flüssigkeitstropfen sind insbesondere bei der Biopolymerpräparation von Flüssigkeitsproben nicht tolerierbar, da hier die Untersuchung von Nukleinsäuren und Proteinen nach vorheriger Durchführung mehrerer (25 bis 40) selbstreplizierender Zyklen, z.B. in der Polymerase Chain Reaction (PCR), durchgeführt wird, mithin sich bereits geringfügig Kontaminationen (Verunreinigungen von 1:1000) vervielfachen und somit zu fehlerhaften Ergebnissen bei der anschließenden Analyse führen.

Die oben beschriebenen Nachteile lassen sich mit den aus dem Stand der Technik bekannten Vorrichtungen nicht in befriedigender Weise vermeiden. Es hat sich nämlich herausgestellt, das im Falle Flüssigkeiten oder wässerige Lösungen, wie z. B. Pufferlösungen, die durch die aus dem Stand der Technik bekannten Vorrichtung - z. B. mittels Vakuum gesaugt werden - eine Kontamination nicht immer vermieden werden kann.

Wird zum Beispiel ein Elutionspuffer durch eine Membran durch Anlegen eines niedrigen Unterdrucks (Restdruck 800 m bar) gesaugt, dann verbleibt in bis zu 20% eine voluminöser Tropfen am Ausgang (Nozzle) des Elutionsgefäßes hängen. Bei den anschließenden Arbeitsschritten besteht somit die Gefahr des Abfallens des Tropfens, beispielsweise in benachbarte Gefäße - womit eine unerwünschte Kreuzkontamination verbunden wäre.

Wird auf der anderen Seite der Elutionspuffer durch Anlegen eines relativ hohen Unterdrucks aus dem Vorratsgefäß herausgesaugt, dann verbleibt in der Regel nur ein kleiner Tropfen am Ausgangsnozzle. Damit ist die Gefahr des Herunterfallens von Tropfen reduziert. Auf der anderen Seite erwächst bei dieser Art der Elution die Gefahr der Kreuzkontamination durch Aerosolbildung. Daneben besiedeln zahlreiche kleine Tropfen die Gefäßwand des Auffanggefäßes, welche sich nur umständlich konzentrieren lassen. Dieser Umstand hat zur Folge das bei beiden Elutionsverfahren die Elutionsvolumina uneinheitlich sind.

Die Aufgabe der vorliegenden Erfindung besteht demgemäß darin, eine möglichst vollständige Elution mit reproduzierbaren Elutionsvolumina zu ermöglichen und die Kontamination anderer Proben durch Analyseflüssigkeiten zu vermeiden.

Gelöst wird diese Aufgabe durch die Verwendung von Kohlenwasserstoffen zur kontaminationsfreien Reinigung oder Trennung von Biopolymeren, welche durch die Zugabe mindestens eines mit Wasser nicht mischbaren Alkans zu den zur Elution oder Filtration eingesetzten wässrigen Lösungen, deren einer Bestandteil das bzw. die Biopolymere sind, gekennzeichnet ist. In einer altemativen Ausführungsform können die zu analysierenden wässerigen Mischungen mit substituierten Alkanen versetzt werden. Diese Alkane können ggf. Substituenten - wie z. B. ein oder mehrere Halogenatom(e), Nitrogruppe(n) oder Aminogruppen(n) - tragen. Voraussetzung der Verwendung von substituierten Alkanen ist, daß sie mit Wasser nicht mischbar sind.

Unter Biopolymeren werden im Sinne der vorliegenden Erfindung natürlich vorkommende Makromoleküle - wie Nucleinsäuren, Proteine oder Polysaccharide - wie auch synthetisch hergestellte - wie z. B. in Fermentationsprozessen erzeugte - Polymere, die gleiche oder ähnliche Bausteine enthalten, wie die natürlichen Makromoleküle, verstanden.

Unter Kohlenwasserstoff im Sinne der Erfindung werden in erster Linie verzweigte oder unverzweigte, substituierte oder substituierte, acyclische oder cyclische Alkane mit 5 bis 20 Kohlenstoffatomen verstanden. Bevorzugt werden verzweigte oder unverzweigte substituierte acyclische oder cyclische Alkane mit 6 bis 16 Kohlenstoffatomen.

Besonders bevorzugt werden unsubstituierte, acyclische verzweigte oder unverzweigte Alkane mit 8 bis 12 Kohlenstoffatomen, worunter n-Octan, n-Nonan, n-Decan und n-Dodecan ganz besonders bevorzugt werden.

Ferner werden Mineralöle ganz besonders bevorzugt, wobei unter Mineralölen im Sinne der vorliegenden Erfindung die aus mineralischen Rohstoffen - wie Erdöl, Braun- und Steinkohlen, Holz oder Torf - gewonnen flüssigen Destillationsprodukte verstanden werden, die im wesentlichen aus Gemischen von gesättigten Kohlenwasserstoffen bestehen [vgl. Römpp, Lexikon Chemie, Thieme Verlag, Stuttgart].

Die Erfindung soll durch die nachfolgenden Beispiele erläutert werden:

### Beispiele

### A Vergleichsbeispiele

Ausgangsmaterial für alle nachfolgenden Experimente bilden eine kommerziell erhältliche Multiwell Filtrationsplatten (96-Well Filterplatte, wie z.B. QIAplate der Firma QIAGEN GmbH, auf der vorab Virus-Präparation durchgeführt wurden oder ungebrauchten Status'). Folgende Elutionsexperimente wurden durchgeführt:
1. Durchsaugen von 80 µl Elutionspuffer (Wasser) bei einem Druck von 800 mbar über einen Zeitraum von 1 Minute. Danach bleibt die Platte noch über einen Zeitraum von 2 Minuten stehen. An der Unterseite der Platte ist danach eine ausgeprägte Tropfenbildung zu beobachten.
   Ein identisches Ergebnis wird bei einem Puffervolumen von 200 µl bei einem Druck von 800 mbar und 2 Minuten Elutionszeit erhalten. Bei der Durchführung der Versuche bei einem Druck von 500, 400 und 200 mbar werden außerdem die Wände der Sammelgefäße (CTMs) mit Analyseflüssigkeit benetzt.
2. Bestimmung der Elutionsvolumina. Durch eine handelsübliche Filterplatte (96 Well) werden bei einem Druck von 800 bar 100 µl Wasser gesaugt. Es werden folgende Volumina erhalten:

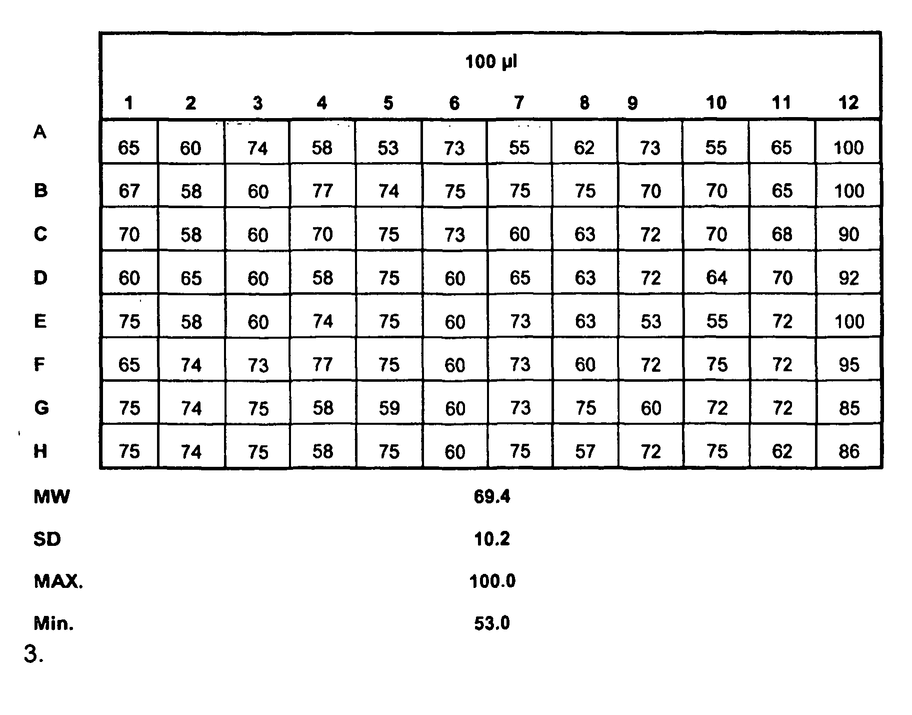

Die visuelle Auswertung des Experiments zeigt, daß an den Auslaßöffnungen eine deutliche Tropfenbildung erkennbar ist. Die erzielten Elutionsvolumina streuen über einen weiten Bereich.

### B Erfindungsgemäße Beispiele

Die unter 1 beschriebenen Versuche werden bei einem Druck von 200 mbar und mit 75 µl Wasser als Elutionspuffer und unter Zugabe von 20 µl n-Octan, n-Nonan, n-Dekan sowie Mineralöl wiederholt.

Dabei ist in allen Fällen weder eine Benetzung der Innenwände der Auffanggefäße mit Wassertropfen noch eine Tropfenbildung an der Platte bzw. an den Auslaßöffnungen zu beobachten.

Daneben werden gleichmäßige Mengen an Eluat erhalten, wie der nachfolgende Vergleichsversuch belegt:

| Wässeriges Eluatvolumen | | | |
|---|---|---|---|
| add 90µl ohne Öl | | add 85 µl plus 30 µl Öl | |
| 67 | 66 | 69 | 69. |
| | | | 5 |
| 54 | 65 | 70 | 70 |
| 65 | 63 | 69 | 71 |
| 67 | 63 | 70 | 70 |
| 30 | 55 | 70 | 67 |
| 32 | 30 | 70 | 70 |
| 30 | 30 | 65 | 70 |
| 64 | 50 | 68 | 74 |

| MW (µl) | | MW (µl) | |
|---|---|---|---|
| | | | |
| 51.9 | | 69.5 | |
| | | | |
| SD | | SD | |
| 15.8 | | 1.9 | |

Dieser Vergleich zeigt folgendes Ergebnis:
1. Dicke Topfen Eluat hängen am Nozzle für die Elution ohne Öl. Zum Teil reißen die Tropfen beim Abnehmen der Platte ab (Kreuzkontamination)
2. Für die Elution mit Öl hängt ein dünner Ölfilm an den Nozzeln. Der Ölfilm verbleibt am Nozzle.
3. Das Eluatvolumen für die Elution mit Öl ist um 20 µl gößer.
4. für die Elution mit Öl ist das Eluatvolumen gleichmäßiger (SD 1.9 versus 15.8).

## Patentansprüche

1. Verwendung von Kohlenwasserstoffen zur kontaminationsfreien Reinigung oder Trennung von Biopolymeren, **dadurch gekennzeichnet, dass** die zur Elution oder Filtration eingesetzten wässrigen Lösungen mit mindestens einem mit Wasser nicht mischbaren Alkan versetzt werden.

2. Verwendung nach Anspruch 1, **dadurch gekennzeichnet, daß** das Alkan unsubstituiert ist.

3. Verwendung nach Anspruch 1, **dadurch gekennzeichnet, daß** das Alkan substituiert ist.

4. Verwendung nach einem der Ansprüche 1 bis 3, **dadurch gekennzeichnet, daß** das Alkan acyclisch ist.

5. Verwendung nach Anspruch 4, **dadurch gekennzeichnet, daß** das acyclische Alkan unverzweigt ist.

6. Verwendung nach Anspruch 4, **dadurch gekennzeichnet, daß** das acyclische Alkan verzweigt ist.

7. Verwendung nach einem der Ansprüche 1 bis 3, **dadurch gekennzeichnet, daß** das Alkan cyclisch ist.

8. Verwendung nach einem der Ansprüche 1 bis 7, **dadurch gekennzeichnet, daß** das Alkan 6 bis 16 Kohlenstoffatome aufweist.

9. Verwendung nach Anspruch 8, **dadurch gekennzeichnet, daß** das Alkan 8 bis 12 Kohlenstoffatome aufweist.

10. Verwendung nach Anspruch 9, **dadurch gekennzeichnet, daß** das Alkan n-Octan, n-Nonan, n-Decan, n-Dodecan ist.

11. Verwendung nach Anspruch 1, **dadurch gekennzeichnet, daß** das Alkan ein Mineralöl ist.

## Claims

1. Use of hydrocarbons for the contamination-free purification or separation of biopolymers, **characterised in that** the aqueous solutions used for the elution or filtration are mixed with at least one alkane, which is not mixable with water.

2. Use according to claim 1, **characterised in that** the alkane is not substituted.

3. Use according to claim 1, **characterised in that** the alkane is substituted.

4. Use according to any one of claims 1 to 3, **characterised in that** the alkane is acyclic.

5. Use according to claim 4, **characterised in that** the acyclic alkane is not branched.

6. Use according to claim 4, **characterised in that** the acyclic alkane is branched.

7. Use according to any one of claims 1 to 3, **characterised in that** the alkane is cyclic.

8. Use according to any one of claims 1 to 7, **characterised in that** the alkane has 6 to 16 carbon atoms.

9. Use according to claim 8, **characterised in that** the alkane has 8 to 12 carbon atoms.

10. Use according to claim 9, **characterised in that** the alkane is n-octane, n-nonane, n-decane, n-dodecane.

11. Use according to claim 1, **characterised in that** the alkane is a mineral oil.

## Revendications

1. Utilisation d'hydrocarbures pour la séparation ou la purification sans contamination de biopolymères, **caractérisée en ce que** l'on ajoute aux solutions aqueuses utilisées pour l'élution ou la filtration au moins un alcane non miscible avec l'eau.

2. Utilisation selon la revendication 1, **caractérisée en ce que** l'alcane est non substitué.

3. Utilisation selon la revendication 1, **caractérisée en ce que** l'alcane est substitué.

4. Utilisation selon l'une des revendications 1 à 3, **caractérisée en ce que** l'alcane est acyclique.

5. Utilisation selon la revendication 4, **caractérisée en ce que** l'alcane acyclique est non ramifié.

6. Utilisation selon la revendication 4, **caractérisée en ce que** l'alcane acyclique est ramifié.

7. Utilisation selon l'une des revendications 1 à 3, **caractérisée en ce que** l'alcane est cyclique.

8. Utilisation selon l'une des revendications 1 à 7, **caractérisée en ce que** l'alcane présente 6 à 16 atomes de carbone.

9. Utilisation selon la revendication 8, **caractérisée en ce que** l'alcane présente 8 à 12 atomes de carbone.

10. Utilisation selon la revendication 9, **caractérisée en ce que** l'alcane est le n-octane, n-nonane, n-décane, n-dodécane.

11. Utilisation selon la revendication 1, **caractérisée en ce que** l'alcane est une huile minérale.
